(19) Europäisches Patentamt
European Patent Office
Office européen des brevets

(11) **EP 2 146 628 B1**

(12) **EUROPÄISCHE PATENTSCHRIFT**

(45) Veröffentlichungstag und Bekanntmachung des Hinweises auf die Patenterteilung:
**09.11.2011 Patentblatt 2011/45**

(51) Int Cl.:
*A61B 5/0205* (2006.01)    *A61B 5/024* (2006.01)
*A61B 5/11* (2006.01)

(21) Anmeldenummer: **08748973.8**

(22) Anmeldetag: **17.04.2008**

(86) Internationale Anmeldenummer:
**PCT/EP2008/003102**

(87) Internationale Veröffentlichungsnummer:
**WO 2008/128709 (30.10.2008 Gazette 2008/44)**

(54) **VORRICHTUNG ZUR ERFASSUNG UND ANALYSE VON VITALPARAMETERN DES KÖRPERS, WIE INSBESONDERE PULS UND ATMUNG**

DEVICE FOR DETECTING AND ANALYZING VITAL PARAMETERS OF THE BODY, SUCH AS PARTICULARLY THE PULSE AND RESPIRATION

DISPOSITIF DE SAISIE ET D'ANALYSE DES PARAMÈTRES VITAUX DE L'ORGANISME, TELS QUE LE POULS ET LA RESPIRATION EN PARTICULIER

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **18.04.2007 DE 102007018677**

(43) Veröffentlichungstag der Anmeldung:
**27.01.2010 Patentblatt 2010/04**

(73) Patentinhaber: **Jäger, Robert**
**75053 Gondelsheim (DE)**

(72) Erfinder: **JÄGER, Robert**
**75053 Gondelsheim (DE)**

(74) Vertreter: **Geitz Truckenmüller Lucht**
**Patentanwälte**
**Kriegsstrasse 234**
**76135 Karlsruhe (DE)**

(56) Entgegenhaltungen:
DE-A1-102005 010 498    GB-A- 2 062 239
US-A- 2 241 190    US-A- 3 944 792
US-A- 4 252 129    US-B1- 6 491 647

- **JAEGER M ET AL: "Respirationsdetektion mittels Mikroklebesensoren" BMT 2006, GEMEINSAME JAHRESTAGUNG DER SCHWEIZERISCHEN, DEUTSCHEN UND ÖSTERREICHISCHEN GESELLSCHAFT FÜR BIOMEDIZINISCHE TECHNIK, 2006, XP002492010 Programmheft Gefunden im Internet: URL:http://www.ssbe.ch/bmt2006/img/program_bmt2006.pdf>**
- **JAEGER M ET AL: "First-aid sensor system: New methods for single-point detection and analysis of vital parameters such as pulse and respiration" ENGINEERING IN MEDICINE AND BIOLOGY SOCIETY, 2007. EMBS 2007. 29TH ANN UAL INTERNATIONAL CONFERENCE OF THE IEEE, IEEE, PI, 1. August 2007 (2007-08-01), Seiten 2928-2931, XP031150099 ISBN: 978-1-4244-0787-3**

**Beschreibung**

Die vorliegende Erfindung betrifft eine Vorrichtung, mit der Vitalparameter des menschlichen Körpers wie Puls und Atmung detektiert und analysiert werden können.

In den Industrieländern sind Erkrankungen im kardiovaskulären System die mit Abstand häufigste Todesursache. Allein in Deutschland sterben daran ca. 44% der Menschen. Einen plötzlichen Herzstillstand, also ein akutes Versagen der Herz-Kreislauffunktion, erleiden ca. zehn Prozent der Bevölkerung. Dabei gibt es verschiedenste Gründe, die zu solch einem kritischen Zustand führen. Ein Herzinfarkt, schwere Verletzungen bei einem Unfall oder ein Stromschlag können zu einer Bewusstlosigkeit führen, die einen Atem- und Herzstillstand verursacht. In solchen Situationen ist es für den Patienten lebenswichtig, dass sein lebensbedrohlicher Zustand möglichst sofort erkannt und er ohne Verzug adäquat reanimiert wird. Mit jeder Minute ohne kardiopulmonale Reanimation (CPR) sinkt die Überlebenswahrscheinlichkeit um zehn Prozent. Zehn Minuten nach einem Kreislaufstillstand gibt es normalerweise keine Überlebenschance mehr.

Um auf solch alltägliche Situationen vorbereitet zu sein, ist es besonders wichtig, dass jeder mit den grundlegenden Maßnahmen in Notfallsituationen mit bewusstlosen Patienten vorbereitet und trainiert ist. Dabei ist es neben der Herz-Lungen-Wiederbelebung besonders wichtig zu wissen, wie das Bewusstsein und die Vtalparameter überprüft werden.

Da es außer beim PKW-Führerschein keine Pflichtveranstaltungen für Erste-Hilfe-Maßnahmen gibt und häufig wesentliches Wissen verloren ist, trauen sich Unfallzeugen nur in seltenen Fällen zu helfen.

Ein weiteres Problem stellen die sich in gewissen Intervallen ändernden Rettungsleitlinien dar. Die letzte Änderung der Leitlinien der "European Resuscitation Council" erfolgte im Jahr 2005. Danach müssen Ersthelfer lediglich das Bewusstsein und die Atmung überprüfen. Vor 2005 musste zusätzlich der Puls durch Tasten an der Halsschlagader überprüft werden. Die Gründe dafür, dass das Überprüfen des Pulses als nicht mehr sinnvoll angesehen wird, sind die fehlende Kompetenz sowie der fehlende Mut der Ersthelfer.

Es gibt bereits einige Vorschläge zur Lösung dieser Problematik.

Das Patent US.4,958,638 Non-contact vital signs monitor" (Sharpe et al., 1990) beschreibt ein System, mit dem gleichzeitig Puls und Atmung gemessen werden können, ohne einen körperlichen Kontakt der Elektroden oder Sensoren zum Körper. Hochfrequenzwellen im Bereich von 10 GHz werden auf die Körperoberfläche gestrahlt und die Laufzeitunterschiede zwischen hin- und rücklaufender Welle gemessen bzw. berechnet. Auf diese Weise lassen sich Informationen über Atem und Puls sowie sämtliche weitere Bewegungen an der Körperoberfläche gewinnen.

Das Patent US 3,993,995 "Respiration Monitor" (Kaplan et al., 1976) beschreibt ein System zur Detektion von Atmung, ohne einen direkten Körperkontakt des Sensors herzustellen. Ein Teil des Brustbereichs wird von Licht bestrahlt. Das reflektierte Licht wird detektiert. Der Phasenunterschied zwischen dem gesendeten und dem reflektierten Licht wird in einem Quadraturdetektor bestimmt, welches indirekt Atembewegungen enthält.

Das Patent US 5,727,549 (Suda et al., 1998) schlägt folgende Methode vor: Durch einen Druck auf ein "Carbon fiber felt" ändert sich dessen Gleichstromwiderstand. Dadurch erfährt ein Konstantstrom durch das Material einen unterschiedlich hohen Widerstand. Wird das System auf die Körperoberfläche geklebt, so erfährt es Druckänderungen durch die von Atmung und Puls hervorgerufenen Körperbewegungen.

Im Patent US 5,273,036 (Kronberg et al., 1993) wird ein Verfahren beschrieben, das als Grundlage die Reflexions-Pulsoxymetrie nutzt. Leuchtdioden senden Licht ins Gewebe. Photodetektoren messen das reflektierte und von Arterien gedämpfte Licht. Dies ändert sich durch die fluktuierenden Pulswellen. Wird der Sensor im Brustbereich adaptiert, so ist die unterschiedliche Intensität auch von der Atembewegung und der hierdurch ausgelösten Kompression des Gewebes und der Blutgefäße abhängig.

Das Patent US 6,823,739 (Ueno et al., 2004) beschreibt ein System, das mittels dünnem Piezomaterial punktuelle Bewegungsdetektion, wie Atmung oder Puls, erkennen kann.

In der Patentschrift US 6,491,647 (Bridger et al.; 2002) wird ein Verfahren beschrieben, mit dem nicht-invasiv und punktuell physiologische Parameter, wie u.a. Puls und Atmung, gemessen werden können. Das System basiert auf mechanischen Veränderungen eines Messaufnehmers, der diese Änderungen in elektrische Signale umwandelt. Der Messaufbau muss dazu auf den Körper, z.B. mittels Brustgurten angepresst werden, damit die Bewegungen an der Hautoberfläche auf den Messaufnehmer übertragen werden.

Im Patent US 6,875,176 (Mourad et al., 2005) wird ein Verfahren beschrieben, bei dem mittels Ultraschall Bewegungen an der Körperoberfläche detektiert werden. Neben dem inneren Schädeldruck können so auch Atmung und Puls erfasst werden.

Das System nach Patent US 6,758,816 (Tsuhata et al., 2004) nutzt den DopplerEffekt für die Feststellung des Pulses. Ein Sender schickt eine Ultraschallwelle in die Arterie, ein Empfänger empfängt das reflektierte Signal.

Alle vorbekannten Systeme zur Erfassung von Puls und/oder Atmung haben sich in der Praxis nicht durchgesetzt, da sie auf einem mehr oder weniger aufwändigen und relativ teuren Verfahren basieren. Bei einigen der beschriebenen Verfahren wird ein Gegendruck benötigt, der durch Gurte hergestellt wird. Solche Systeme sind aufwändig in der Bedienung, weder klein noch laientauglich und daher für den mobilen Erste-Hilfe-Einsatz kaum geeignet. Die bekannten Vorrichtungen benötigen zudem alle relativ viel elektrische Energie, was vor allem für den mobilen Erste-Hilfe-Einsatz nicht akzeptabel ist.

Angesichts der aufgezeigten Nachteile im Stand der Technik ist es Aufgabe der vorliegenden Erfindung, ein System zur Diagnose von Vitalparametern wie Atmung und Puls bereitzustellen, welches schnell und zuverlässig arbeitet und auch von unerfahrenen Ersthelfern am Unfallort eingesetzt werden kann.

Gelöst wird die Aufgabe durch eine Vorrichtung mit den im Anspruch 1 angegebenen Merkmalen.

Die erfindungsgemäße Vorrichtung beruht auf der Idee, kleinste Änderungen in den obersten Körperschichten zu detektieren und daran die Vitalparameter, insbesondere Puls und Atmung, zu erkennen. Das Heben und Senken des Brustkorbs bzw. Bauchs beim Atmen ebenso wie das in den Adern pulsierende Blut führen zu periodischen mechanischen Veränderungen an der Körperoberfläche. Um diese Veränderungen in den obersten Körperschichten zu detektieren, wird ein LC-Schwingkreis benutzt, an den ein metallisches Sensorelement angekoppelt ist. Das Sensorelement wird in unmittelbare Nähe der Körperoberfläche gebracht, ohne dass es diese berührt. Das Sensorelement stellt eine Antenne für elektromagnetische Wellen im Nahbereich dar oder kann in erster Näherung als eine Platte eines Plattenkondensators aufgefasst werden, wobei die benachbarten Körperschichten die andere Platte darstellen. Etwas genauer betrachtet, entspricht die Anordnung allerdings eher einem Kugelkondensator mit dem metallischen Sensorelement in der Mitte und den benachbarten Körperschichten als Dielektrikum. Selbst kleinste Veränderungen in den obersten Körperschichten oder geringste Änderungen des Abstands zwischen Sensorelement und Körperoberfläche bewirken so eine Änderung der Kapazität, wodurch sich die Resonanzfrequenz des LC-Schwingkreises etwas erhöht oder erniedrigt. Die Verstimmung des Resonanzkreises führt zu einem Abfall der Schwingungsamplitude. Dieser Spannungsabfall kann als Gleichspannung gungsamplitude. Dieser Spannungsabfall kann als Gleichspannung sehr einfach gemessen werden. Die am LC-Schwingkreis gemessene Amplitudenspannung stellt somit ein Maß für die mechanischen Veränderungen in den obersten Hautschichten dar. Durch Analyse des Amplitudensignals mithilfe eines Mikroprozessors können die Vitalparameter bestimmt und insbesondere erkannt werden, ob Atmung und/oder Puls vorhanden ist.

Atmung und Puls eines Menschen können am einfachsten am Hals, vorzugsweise im Bereich oberhalb des Schlüsselbeins, erkannt werden. Dabei muss das Sensorelement nicht zwingend über der Arteria Carotis aufgebracht werden, denn aufgrund der Ausbreitung der Pulswelle im umliegenden Gewebe sind die Veränderungen in den obersten Hautschichten auch noch in einiger Entfernung hinreichend groß, um von dem Sensorelement erfasst zu werden. Atembewegungen können bis in den Bereich der Schulter hinein gut registriert werden.

Ein großer Vorteil der Erfindung ist, dass die Vitalparameter Puls und Atmung gemeinsam an einer Stelle des Körpers erfasst werden. Die Anbringung eines einzigen Sensorelements, dass klein und leicht ausgeführt sein kann, an einer gut zugänglichen Stelle des Körpers, wie insbesondere im Bereich des Halses oder Schlüsselbeins, macht das System besonders notfalltauglich und selbst für Laien leicht handhabbar. Ein weiterer großer Vorteil besteht darin, dass das System automatisch arbeitet, indem es das aus den Veränderungen in den obersten Körperschichten gewonnene Signal selbsttätig analysiert und daraus die Vitalparameter bestimmt. Der Anwender muss also weder Erfahrung im Fühlen des Pulses haben noch in der Beobachtung der Atmung geschult sein. Die wichtigste Frage, die sich ein Ersthelfer stellt, nämlich, ob der Patient wiederbelebt werden muss, wird vom Gerät schnell und klar beantwortet. Hierzu stehen heute selbstlernende Verfahren und Algorithmenzur Verfügung, mit denen die detektierten Signale analysiert und ausgewertet werden können. Im einfachsten Fall kann die Auswerteinheit eine unmissverständliche Information, wie zum Beispiel "Puls und Atmung vorhanden" oder "Sofort reanimieren!" ausgeben.

Das System arbeitet umso genauer und zuverlässiger, je empfindlicher der LC-Schwingkreis auf eine Beeinflussung seiner Resonanzfrequenz infolge kapazitiver, induktiver oder auch dämpfender Effekte reagiert. Bevorzugt wird deshalb ein LC-Schwingkreis eingesetzt, der eine nichtlineare Charakteristik mit sehr steiler Resonanzkurve aufweist. Im Gegensatz zu einem linearen Schwingkreis führen bei einem nichtlinearen Schwingkreis mit sehr steiler oder gar überhängender Resonanzkurve schon kleine oder kleinste Veränderung der Induktivität oder Kapazität zu einer überproportional großen Änderung der Schwingungsamplitude. Selbst kleinste Veränderungen in den obersten Hautschichten lassen sich so zuverlässig detektieren. Ein weiterer Vorteil bei Verwendung eines nichtlinearen LC-Schwingkreises ist der sehr geringe Energieverbrauch des hochempfindlichen Messsystems. Die gesamte Vorrichtung lässt sich dadurch sehr klein und auch kostengünstig bauen, was für einen mobilen Einsatz im Rettungswesen wichtig ist. Weitere vorteilhafte und zweckmäßige Ausgestaltungen der erfindungsgemäßen Vorrichtung ergeben sich aus den Unteransprüchen.

Ausführungsbeispiele der Erfindung werden anhand der beigefügten Abbildungen näher beschrieben. Es zeigen:

Figur 1          ein Gerät zur Bestimmung der Vitalparameter, stark vereinfacht;

Figur 2          ein Blockschaltbild des Geräts von Fig. 1;

Figur 3a         die Resonanzkurve eines linearen LC-Schwingkreises;

Figur 3b          die Resonanzkurve eines nichtlinearen LC-Schwingkreises;

Figuren 4a, 4b, 4c    Prinzipschaltbilder des nichtlinearen LC-Schwingkreises mit und ohne Last;

Figur 5          die Resonanzkurve des nichtlinearen LC-Schwingkreises mit und ohne ohmschen Verlustwiderstand;

Figur 6          das Prinzipschaltbild des nichtlinearen LC-Schwingkreises mit angeschlossenem Sensorelement;

Figur 7          ein Kondensator-Prinzipschaltbild des an die Körperoberfläche gebrachten Sensorelements;

Figur 8          die Wirbelstrom-Effekte im Bereich des Sensorelements;

Figur 9          ein Ablaufdiagramm für die Signalverarbeitung;

Figur 10         ein Flussdiagramm für das ganz Gerät;

Figur 11         ein Amplituden-Zeit-Diagramm für das gemessene Rohsignal und die daraus extrahierten Signale für Puls und Atmung.

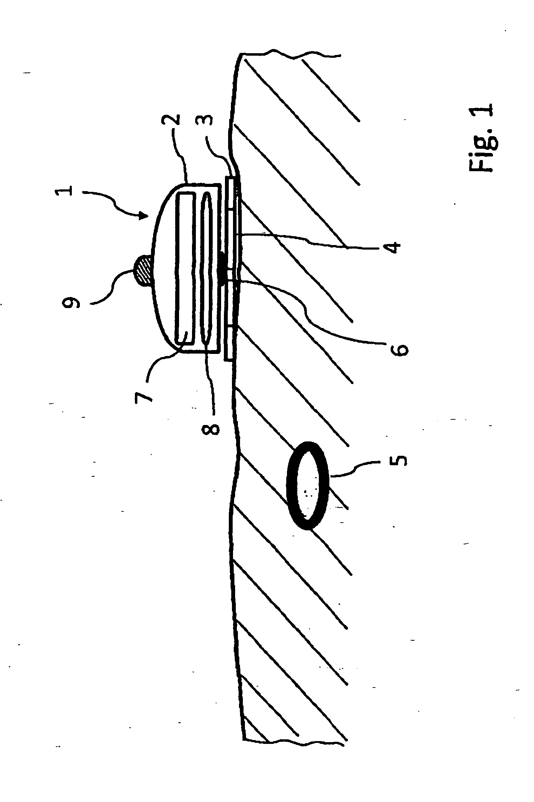

In der Figur 1 ist ein speziell für den Erste-Hilfe-Einsatz vorgesehenes Gerät 1 zu sehen. Alle elektronischen Komponenten sind in einem kleinen runden Gehäuse 2 untergebracht. An der Unterseite befindet sich ein Sensorelement in Form einer dünnen Metallplatte 3, die elektrisch gut leitfähig ist. An seiner Vorderseite (in der Abbildung unten) ist die Metallplatte 3 mit einer selbstklebenden Schicht 4 versehen, die gleichzeitig eine elektrische Isolierschicht und die Funktion eines Abstandshalters hat. Mittels der Selbstklebeschicht 4 wird das Gerät 1 auf der Haut des Patienten fixiert, und zwar in der Nähe der Arteria Carotis 5. Die Dicke der isolierenden Schicht beträgt ca. 0,1 mm und definiert damit den Abstand zwischen Metallplatte 3 und Körperoberfläche. Auf der Rückseite (in der Abbildung oben) trägt die Metallplatte 3 einen Steckanschluss 6, der im Prinzip wie ein Druckknopf funktioniert und der mechanischen und elektrischen Verbindung mit den im Gehäuse 2 eingebauten Komponenten des Geräts dient.

Im Inneren des Gehäuses 2 befinden sich eine hochintegrierte elektronische Schaltung 7 und eine Energieversorgung in Form einer Batterie 8. Das Ergebnis der Diagnose wird als optisches Signal auf einer Anzeige 9 ausgegeben, die außen auf dem Gehäuse 2 angebracht ist.

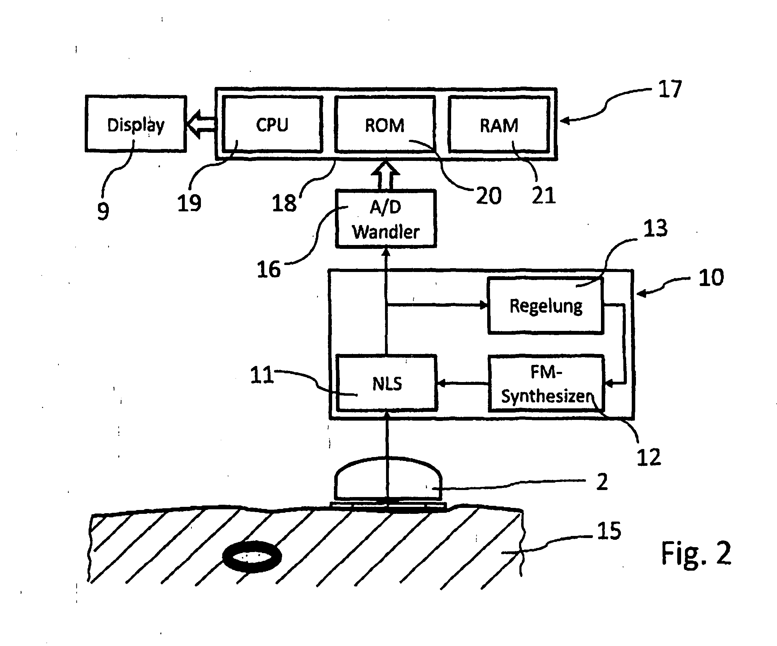

Das Blockschaltbild von Figur 2 lässt den grundsätzlichen Aufbau der elektronischen Schaltung 7 erkennen. Sie umfasst eine Signalerfassungseinheit 10 mit einem nichtlinearen LC-Schwingkreis (NLS) 11, der von einem Frequenzgenerator 12 erregt wird. Eine Frequenzregelung 13 hält die eingestellte Schwingungsfrequenz konstant. Die in geringem Abstand (ca. 0,1 mm) über der Körperoberfläche 14 fixierte Metallplatte 3 ist derart an den LC-Schwingkreis 11 angekoppelt, dass kleine und kleinste Bewegungen bzw. mechanische Veränderungen in den obersten Körperschichten 15 eine Verschiebung der Resonanzfrequenz des LC-Schwingkreises 11 bewirken. Die dadurch bedingte Änderung der Schwingungsamplitude des LC-Schwingkreises 11 (bei unveränderter Erregung) wird gemessen und steht als analoges Spannungssignal am Ausgang der Signalerfassungseinheit 10 zur Verfügung.

Ein Analog/Digital-Wandler 16 formt das analoge Amplitudensignal in ein entsprechendes Digitalsignal um. Das digitalisierte Amplitudensignal wird einer Auswerteinheit 17 zugeführt, welche aus einem Mikroprozessor 18 mit CPU 19, nicht flüchtigem ROM-Speicher 20 und RAM-Arbeitsspeicher 21 besteht. Der Mikroprozessor 18 analysiert das Amplitudensignal und bestimmt anhand abgespeicherter Kriterien, ob der Patient genügend atmet und/oder einen ausreichend kräftigen Puls hat. Das Ergebnis der Analyse wird als optisches und/oder akustisches Signal ausgeben. Hierzu dient die Anzeige 9 oben auf dem Gehäuse 2 (vgl. Figur 1).

Der hier verwendete LC-Schwingkreis umfasst außer einer Induktivität (L) eine nichtlineare Kapazitätsdiode, also eine spannungsgesteuerte Kapazität (C) mit Richtungswirkung. Mit einem solchen nichtlinearen Schwingkreis lassen sich sehr steile Resonanzkurven einstellen. In der Paterttanmeldung DE 10 2005 010 498 desselben Anmelders sind die Funktionsweise und Eigenschaften eines nichtlinearen Schwingkreises näher beschrieben.

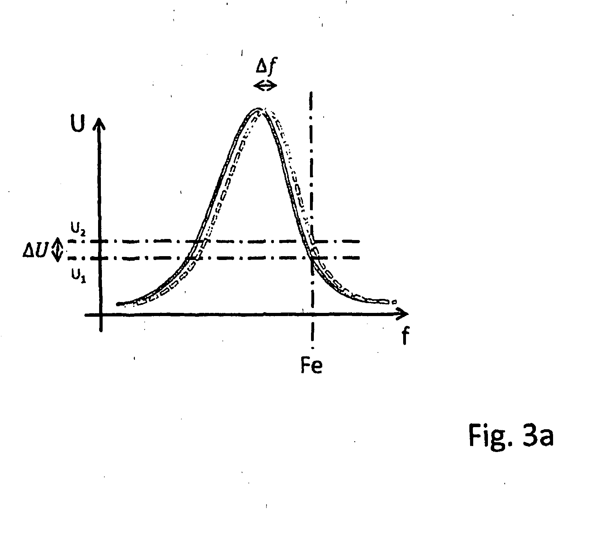

Figur 3a veranschaulicht die Resonanzkurve eines LC-Schwingkreises mit linearer Charakteristik. Aufgetragen ist die Schwingungsamplitude U über der Frequenz f. Veränderungen der Induktivität oder der Kapazität führen zu einer Verschiebung der Resonanzfrequenz zu höheren oder niedrigeren Frequenzen hin. Die bei diesem Beispiel nach rechts verschobene Resonanzkurve ist gestrichelt dargestellt. Wird der Schwingkreis mit einer Frequenz Fe erregt, führt eine Verschiebung der Resonanzfrequenz um Δf zu einem ungefähr proportionalen Abfall der Schwingungsamplitude ΔU.

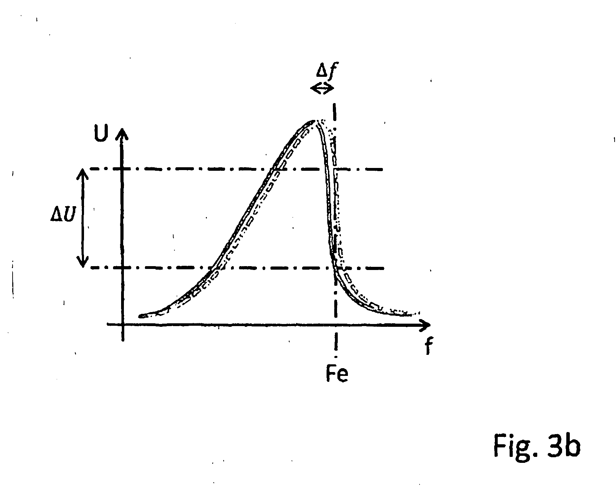

Figur 3b zeigt dagegen die Resonanzkurve eines nichtlinearen LC-Schwingkreises. Auffällig ist der sehr steile, fast schon vertikale Verlauf des rechten Astes der Resonanzkurve. Stellt man die Erregerfrequenz Fe so ein, dass sie im Bereich dieses steilen Kurvenastes liegt, so führt bereits eine kleine Verschiebung der Resonanzfrequenz Δf zu einem überproportionalen Abfall der Schwingungsamplitude ΔU. Somit können mit einem nichtlinearen LC-Schwingkreis ohne großen schaltungstechnischen Aufwand extrem hohe Amplitudenänderungen erzeugt werden. Selbst mit kleinsten Änderungen der Resonanzfrequenz lassen sich nahezu beliebig große Änderungen in der Amplitude realisieren. Im Extremfall lässt sich sogar eine Resonanzkurve mit senkrechter Flanke oder gar eine überhängende Resonanzkurve erzeugen, sodass das System in einem bestimmten Frequenzbereich leicht kippt.

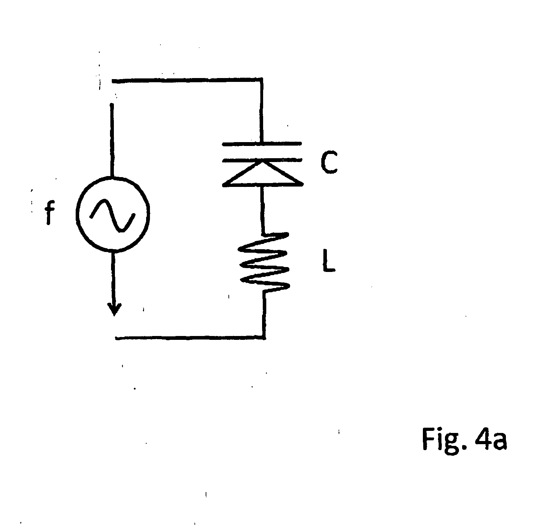

In Figur 4a ist das Prinzipschaltbild des nichtlinearen LC-Schwingkreises 11 aus der Schaltung nach Figur 2 dargestellt. Der Schwingkreis enthält neben der Induktivität L eine Varaktordiode mit der Kapazität C. Die Resonanzfrequenz berechnet sich nach der Formel

$$f_0 = \frac{1}{2\pi\sqrt{LC}}$$

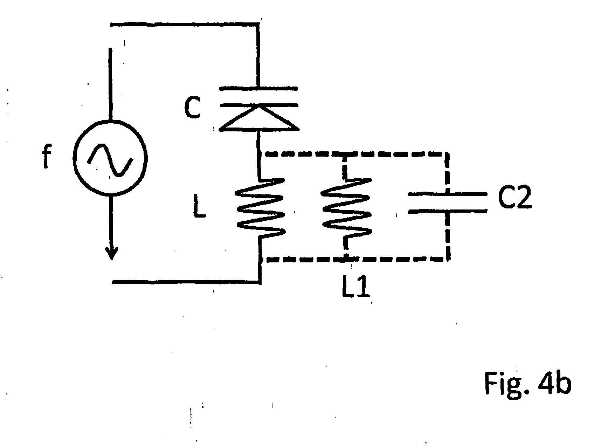

-In Figur 4b sind parallel zur Induktivität L des Schwingkreises eine weitere Induktivität L1 und eine zusätzliche Kapazität C1 geschaltet. Hierdurch ändert sich die Resonanzfrequenz des LC-Schwingkreises.

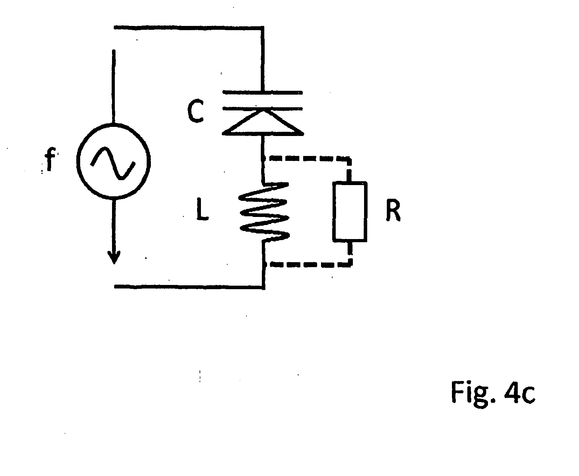

Gemäß Figur 4c können neben rein induktiven oder kapazitiven Bauteilen auch reelle Widerstände R hinzugefügt werden. Deren ohmsche Verluste beeinflussen allerdings nicht die Resonanzfrequenz des Schwingkreises, sondern nur die Höhe und Breite der Resonanzkurve.

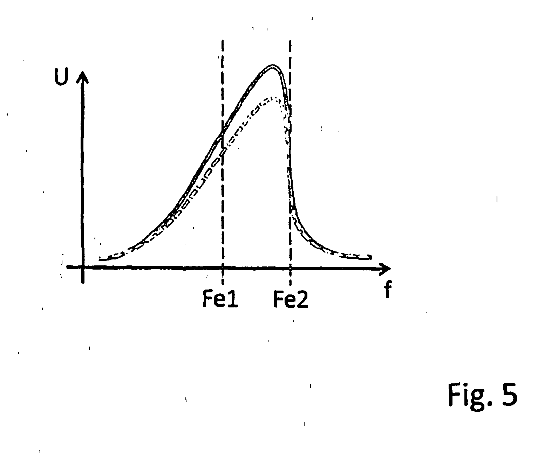

In Figur 5 ist die typische Resonanzkurve eines nichtlinearen LC-Schwingkreises einmal ohne und (gestrichelt) mit zusätzlichem ohmschen Verlustwiderstand dargestellt. Liegt die Erregerfrequenz Fe1 an einer weniger steilen Stellen der Resonanzkurve, bewirken ohmsche Verluste einen relativ großen Abfall der Schwingungsamplitude. Liegt die Erregerfrequenz Fe2 in einem sehr steilen Bereich der Resonanzkurve, fällt der gleiche ohmsche Verlust weniger stark ins Gewicht gegenüber dem viel größeren Einbruch der Amplitude infolge einer Änderung der Kapazität und/oder Induktivität. Trotzdem ist darauf zu achten, dass die Resonanzkurve durch zu große ohmsche Verluste nicht völlig abflacht.

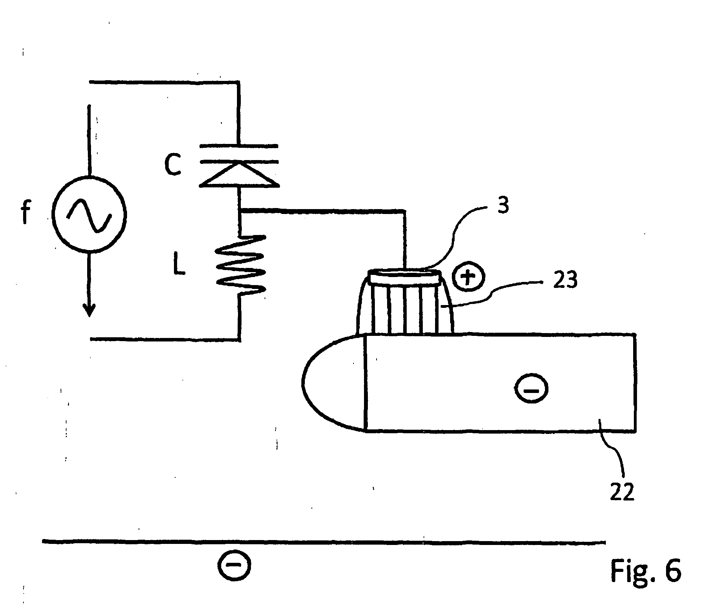

Figur 6 zeigt das Prinzipschaltbild des nichtlinearen LC-Schwingkreises mit der zwischen Induktivität L und Kapazität C als Sensorelement angeschlossenen Metallplatte 3 (vgl. Fig. 1 und Fig. 2), die auf Veränderungen des Körpergewebes 22 anspricht. Bei genügend hoher Frequenz wirkt die Metallplatte 3 als Antenne für elektromagnetische Wellen im Nahbereich und stellt, stark vereinfacht, gleichzeitig die eine Platte eines Plattenkondensators dar, dessen andere Platte das Körpergewebe 22 ist. Bei genügender Annäherung an das Körpergewebe 22 bilden sich gerichtete Feldlinien 23 aus. Eine Änderung des Abstands zwischen Metallplatte 3 und Körpergewebe 22 führt zu einer Kapazitätsänderung, ebenso Veränderungen im Körpergewebe 22 selbst.

Im Gegensatz zu einem Kondensator sind die elektrischen Parameter des Körpergewebes allerdings nicht eindeutig zu beschreiben. Dies hängt damit zusammen, dass die Körperoberfläche aus unterschiedlichen Gewebeschichten aufgebaut ist. Da diese Gewebeschichten nur relativ schwach elektrisch leitend sind, dringen die von der Metallplatte 3 ausgestrahlten elektromagnetischen Wellen zumindest in die obersten Schichten ein und treffen dabei sogar auf Adern, in denen Blut fließt.

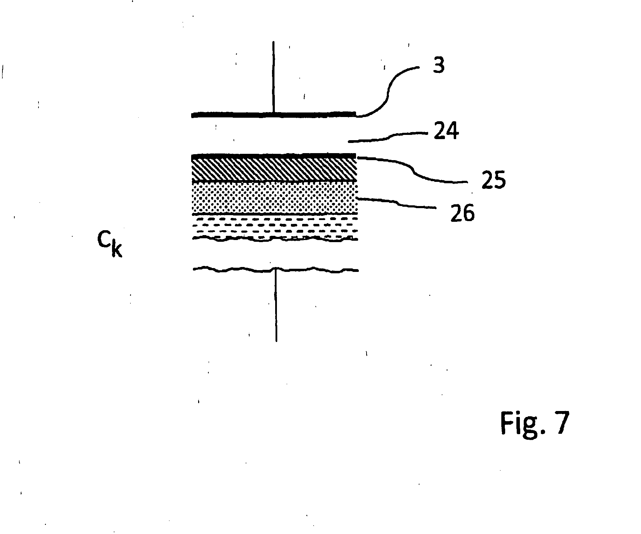

Die obersten Hautschichten und der Zwischenraum zwischen der isolierten Metallplatte 3 und der Körperoberfläche lassen sich auch als Kondensator mit mehreren hintereinander geschalteten Schichten mit unterschiedlichen Dielektrizitätskonstanten darstellen. Figur 7 zeigt ein entsprechendes Prinzipschaltbild, umfassend die als Sensorelement dienende Metallplatte 3, den Zwischenraum 24 zwischen Metallplatte 3 und Hautoberfläche 25 sowie die unmittelbar unterhalb der Hautoberfläche 25 liegenden Hautschichten 26. Jede dieser Schichten hat eine andere Dielektrizitätskonstante, die in die Gesamtkapazität Ck eingehen. Veränderungen, insbesondere Ausdehnungen der einzelnen Hautschichten 26 sowie eine Veränderung des Abstands zwischen Metallplatte 3 und Hautoberfläche 25 führen zu einer Änderung der Kapazität Ck und damit auch zu einer Änderung der Gesamtkapazität im Schwingkreis, die zu einer Erhöhung oder Erniedrigung dessen Resonanzfrequenz führt.

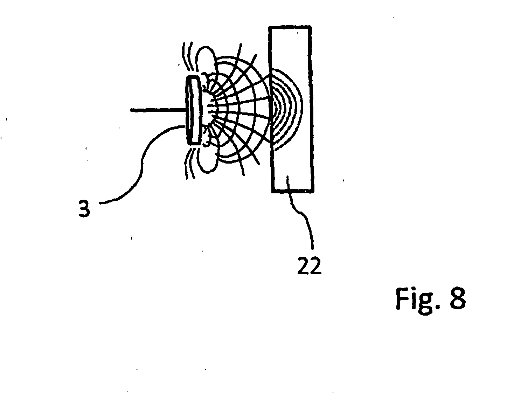

Figur 8 veranschaulicht, wie durch Wirbelstrom-Effekte im Körpergewebe 22 die ohmschen Verluste im LC-Schwingkreis beeinflusst werden. Diese wirken sich auf Höhe und Breite der Resonanzkurve aus. Induktive Effekte können dagegen vernachlässigt werden.

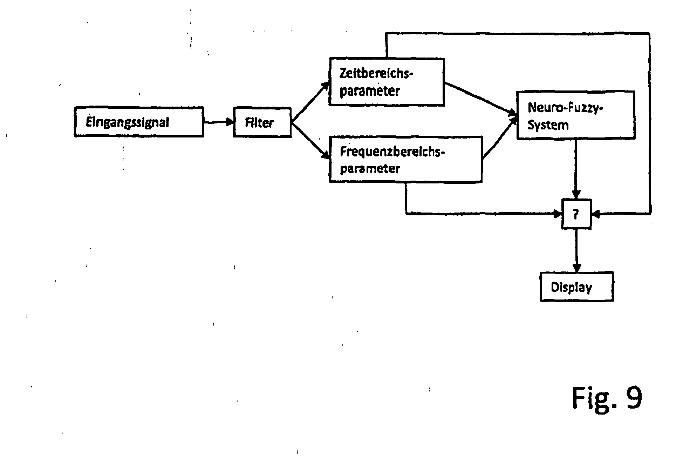

Das von der Signalerfassungseinheit 10 (vgl. Figur 2) bereitgestellte Amplitudensignal muss in der Auswerteinheit 17 so weiterverarbeitet werden, dass ein auch dem Laienhelfer verständliches Ergebnis angezeigt wird. Der grobe Ablauf ist in Figur 9 skizziert.

Das digitale Eingangssignal wird einem selbstregulierenden Filter zugeführt. Anschließend erfolgen eine Extraktion typischer Merkmale im Zeit- und Frequenzbereich und eine Auswertung durch ein selbstlernendes Neuro-Fuzzy-System. Schließlich werden sämtliche Parameter in einer Entscheidungseinheit zusammengeführt und die dort generierte Entscheidung an ein Display übermittelt. Alle Teilergebnisse der einzelnen Rechenschritte sowie das Endergebnis werden mit Zeitstempel gespeichert. Die Entscheidungsfindung erfolgt anhand von Ärzten empfohlenen Kriterien. Durch die Verwendung eines neuronalen Fuzzy-Logik-Netzwerks ist es möglich, auch differenzierte Ergebnisse zuzulassen wie zum Beispiel "Eventuell Atemstillstand" oder" Sehr schwacher Puls".

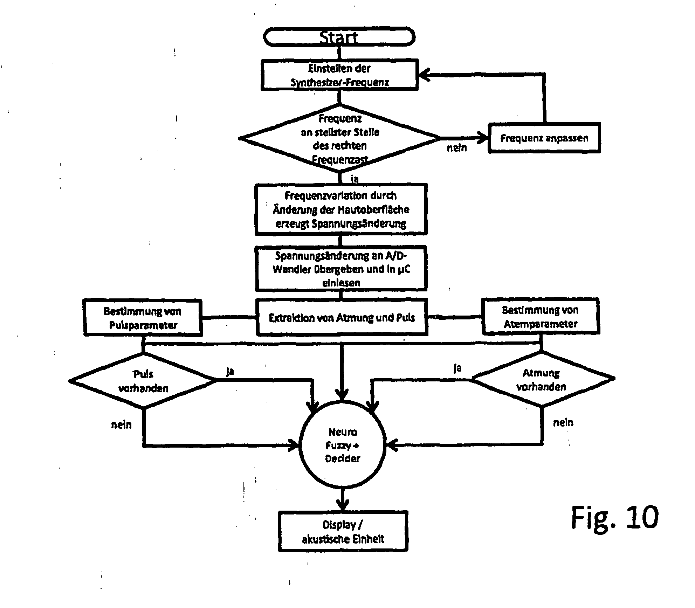

Das Flussdiagramm von Figur 10 gibt den gesamten Ablauf beim Einsatz des Geräts wieder.

Lediglich die Anbringung des Geräts durch Aufkleben des Sensorelements auf die Körperoberfläche muss noch manuell geschehen. Ab diesem Moment laufen dann alle Schritte, also die Erfassung und Analyse der Vitalparameter bis hin zur Ausgabe eines Ergebnissignals automatisch ab. Der Helfer sollte nur darauf achten, dass sich der Patient nicht zuviel bewegt.

Sofort nach dem Aufkleben des Sensorelements werden zuerst die Kapazitäten, Induktiven und ohmschen Verluste im System festgestellt und basierend auf diesen Werten die Erregerfrequenz für den LC-Schwingkreis auf einen optimalen Wert eingestellt, das heißt auf die steilste Stelle der Resonanzkurve. Ist die optimale Einstellung gefunden, muss im Folgenden nur noch überwacht werden, ob die Ist-Frequenz über einen längeren Zeitraum von der Soll-Frequenz abweicht. Gegebenenfalls wird die Ist-Frequenz nachgeregelt bzw. an veränderte Bedingungen angepasst. Damit ist eine gleich bleibende hohe Empfindlichkeit und Messgenauigkeit gewährleistet.

Die durch Atmung und/oder Puls hervorgerufenen Veränderungen in den obersten Hautschichten führen zur Verstimmung des Schwingkreises, die wiederum eine Änderung der Schwingungsamplitude bewirkt. Diese Spannungsänderungen werden mittels A/D-Wandler digitalisiert und an den Mikroprozessor übergeben. Dort wird das Rohsignal, das alle erfassten Körperparameter umfasst, in die Bereiche "Atmung" und "Puls" aufgeteilt. Mit speziellen Algorithmen werden die Parameter und die Qualität des Pulses und der Atmung getrennt bestimmt. Alle Informationen wie z.B. "Atmung oder Puls vorhanden" bzw. "nicht vorhanden" werden schließlich in einer selbstlernenden Fuzzy-Logik miteinander verknüpft und bewertet. Das daraus gewonnene Gesamturteil wird auf einem Display im Klartext und/oder als akustisches Signal ausgegeben.

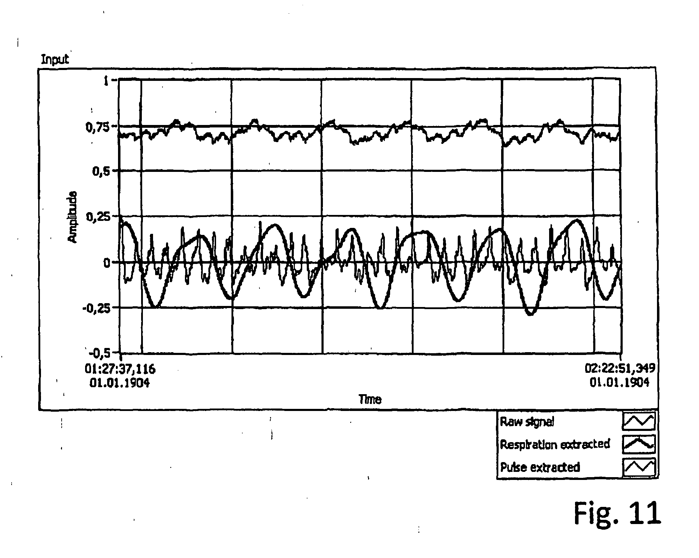

Figur 11 veranschaulicht, wie aus dem gemessenen Rohsignal (oben) durch den Computer die Signale für Puls und Atmung (unten) extrahiert werden. Das Pulssignal hat hier eine viel höhere Frequenz als das Atemsignal, was ein Indiz dafür ist, dass der Patient atmet und normalen Puls hat. Die getrennte Weiterverarbeitung der extrahierten Signale erfolgt mittels spezifischer Algorithmen.

Bezugszeichen

| 1 | Gerät |
|---|---|
| 2 | Gehäuse |
| 3 | Metallplatte |
| 4 | Selbstklebende Schicht |
| 5 | Arteria carotis |
| 6 | Steckanschluss |
| 7 | Elektronische Schaltung |
| 8 | Batterie |
| 9 | Anzeige |
| 10 | Signalerfassungseinheit |
| 11 | LC-Schwingkreis |
| 12 | Frequenzgenerator |
| 13 | Frequenzregelung |
| 14 | Körperoberfläche |

15    Körperschichten

16    A/D-Wandler

17    Auswerteinheit

18    Mikroprozessor

19    CPU

20    ROM-Speicher

21    RAM-Speicher

22    Körpergewebe

23    Feldlinien

24    Zwischenraum

25    Hautoberfläche.

26    Hautschichten

**Patentansprüche**

1.    Vorrichtung zur Erfassung und Analyse von Vitalparametern des Körpers, wie insbesondere Puls und Atmung, mit

   - einem LC-Schwingkreis (11), der zu Schwingungen im Bereich seiner Resonanzfrequenz angeregt wird;
   - einem metallischen Sensorelement, das in geringem Abstand zur Körperoberfläche fixierbar ist und an den LC-Schwingkreis (11) derart angekoppelt ist, dass kleine Veränderungen in den obersten Körperschichten (15) eine Verschiebung der Resonanzfrequenz bewirken, wodurch es zu einer Änderung der Schwingungsamplitude kommt;
   - einer Signalerfassungseinheit (10), welche die Schwingungsamplitude des LC-Schwingkreises (11) mißt und als Amplitudensignal zur Verfügung stellt;
   - einer Auswerteinheit (17) mit Mikroprozessor (18), der das Amplitudensignal analysiert und bewertet, um die Vitalparameter zu bestimmen,
   **dadurch gekennzeichnet, dass** es sich bei dem Sensorelement um eine mit einer selbstklebenden Isolierschicht versehene Metallplatte (3) zur Befestigung auf der Haut handelt und der LC-Schwingkreis (11) eine nichtlineare Charakteristik mit einer Resonanzkurve aufweist, welche an ihrem rechten Ast einen zumindest annähernd vertikalen Verlauf annimmt.

2.    Vorrichtung nach Anspruch 1, **dadurch gekennzeichnet, dass** der LC-Schwingkreis eine nichtlineare Kapazitätsdiode umfasst.

3.    Vorrichtung nach einem der Ansprüche 1 oder 2, **dadurch gekennzeichnet, dass** der LC-Schwingkreis (11) durch einen Frequenzgenerator (12) mit regelbarer Frequenz erregt wird.

4.    Vorrichtung nach einem der vorhergehenden Ansprüche,
   **dadurch gekennzeichnet, dass** auf der Vorderseite der Metallplatte (3) ein Abstandshalter aus isolierendem Material angebracht ist.

5.    Vorrichtung nach einem der vorhergehenden Ansprüche,
   **dadurch gekennzeichnet, dass** die Metallplatte (3) auf ihrer Rückseite einen Steckanschluss (6) trägt.

6.    Vorrichtung nach einem der vorhergehenden Ansprüche,
   **dadurch gekennzeichnet, dass** zwischen der Signalerfassungseinheit (10) und der Auswerteinheit (17) ein Analog/Digital-Wandler (16) angeordnet ist, der das analoge Amplitudensignal in ein digitales Signal umwandelt.

7. Vorrichtung nach einem der vorhergehenden Ansprüche, **gekennzeichnet durch** eine Anzeige (9) zur optischen und/oder akustischen Ausgabe des von der Auswerteinheit ermittelten Ergebnisses.

8. Vorrichtung nach Anspruch 7, **dadurch gekennzeichnet, dass** das Sensorelement mit LC-Schwingkreis (11), die Signalerfassungseinheit (10), die Auswerteinheit (17) und die Anzeige (9) in einem gemeinsamen Gehäuse (2) integriert sind.

9. Vorrichtung nach Anspruch 8, **dadurch gekennzeichnet, dass** das Gehäuse (2) eine im Wesentlichen ebene Unterseite aufweist, in deren Bereich das Sensorelement angeordnet ist.

**Claims**

1. Device for detection and analysis of vital parameters of the body, such as, in particular, pulse and respiration, having

    - an LC oscillating circuit (11) that is excited to produce oscillations in the range of its resonant frequency;
    - a metallic sensor element that can be fixed in place at a slight distance from the body surface and is coupled with the LC oscillating circuit (11) in such a manner that small changes in the uppermost body layers (15) bring about a displacement of the resonant frequency, thereby causing a change in the oscillation amplitude;
    - a signal detection unit (10) that measures the oscillation amplitude of the LC oscillating circuit (11), and makes it available as an amplitude signal;
    - an evaluation unit (17) having a microprocessor (18), which analyses and evaluates the amplitude signal, in order to determine the vital parameters,
    **characterised in that** the sensor element is a metal plate (3) provided with a self-adhesive insulation layer for fastening to the skin and the LC oscillating circuit (11) has a non-linear characteristic with a resonance curve, the right branch of which assumes an almost vertical profile.

2. Device according to claim 1, **characterised in that** the LC oscillating circuit (11) comprises a non-linear variable capacitance diode.

3. Device according to one of claims 1 or 2, **characterised in that** the LC oscillating circuit (11) is excited by a frequency generator (12) having a controllable frequency.

4. Device according to one of the preceding claims, **characterised in that** a spacer made of insulating material is affixed to the front of the metal plate (3).

5. Device according to one of the preceding claims, **characterised in that** the metal plate (3) carries a plug connector (6) on its back side.

6. Device according to one of the preceding claims, **characterised in that** an analogue/digital converter (16) is disposed between the signal detection unit (10) and the evaluation unit (17), which converter converts the analogue amplitude signal into a digital signal.

7. Device according to one of the preceding claims, **characterised by** a display (9) for optical and/or acoustic output of the result determined by the evaluation unit.

8. Device according to claim 7, **characterised in that** the sensor element having the LC oscillating circuit (11), the signal detection unit (10), the evaluation unit (17), and the display (9) are integrated into a common housing (2).

9. Device according to claim 8, **characterised in that** the housing (2) has a substantially planar underside, in the region of which the sensor element is disposed.

**Revendications**

1.  Dispositif de détection et d'analyse de paramètres vitaux du corps, tels que le pouls et la respiration en particulier, comprenant

    - un circuit oscillant LC (11) que l'on soumet à une excitation pour qu'il produise des oscillations proches de sa fréquence de résonance ;
    - un élément capteur métallique apte à être fixé à une faible distance par rapport à la surface du corps et qui est couplé au circuit oscillant LC (11) de telle sorte que de petits changements dans les couches supérieures (15) du corps provoquent un décalage de la fréquence de résonance, induisant ainsi une modification de l'amplitude des oscillations ;
    - une unité de détection de signal (10) qui mesure l'amplitude des oscillations du circuit oscillant LC (11) et la fournit sous forme de signal d'amplitude ;
    - une unité d'évaluation (17) avec microprocesseur (18), lequel analyse
    et évalue le signal d'amplitude pour déterminer les paramètres vitaux, **caractérisé en ce que** l'élément capteur est une plaque de métal (3) munie d'une couche isolante autoadhésive, destinée à être fixée sur la peau, et le circuit oscillant LC (11) présente une caractéristique non linéaire avec une courbe de résonance prenant sur sa branche droite une allure au moins approximativement verticale.

2.  Dispositif selon la revendication 1, **caractérisé en ce que** le circuit oscillant LC comprend une diode à capacité variable non linéaire.

3.  Dispositif selon l'une des revendications 1 ou 2, **caractérisé en ce que** le circuit oscillant LC (11) est excité par un générateur de fréquence (12) à fréquence réglable.

4.  Dispositif selon l'une des revendications précédentes, **caractérisé en ce qu'**un espaceur en matière isolante est monté sur la face avant de la plaque de métal (3).

5.  Dispositif selon l'une des revendications précédentes, **caractérisé en ce que** la plaque de métal (3) porte sur sa face arrière un connecteur à fiche (6).

6.  Dispositif selon l'une des revendications précédentes, **caractérisé en ce qu'**un convertisseur analogique-numérique (16) est disposé entre l'unité de détection de signal (10) et l'unité d'évaluation (17), lequel convertit le signal d'amplitude analogique en signal numérique.

7.  Dispositif selon l'une des revendications précédentes, **caractérisé par** un indicateur (9) de sortie optique et/ou acoustique du résultat déterminé par l'unité d'évaluation.

8.  Dispositif selon la revendication 7, **caractérisé en ce que** l'élément capteur avec circuit oscillant LC (11), l'unité de détection de signal (10), l'unité d'évaluation (17) et l'indicateur (9) sont intégrés dans un boîtier (2) commun.

9.  Dispositif selon la revendication 8, **caractérisé en ce que** le boîtier (2) a une face inférieure substantiellement plane, dans la zone de laquelle est disposé l'élément capteur.

Fig. 1

Fig. 2

EP 2 146 628 B1

Fig. 3a

Fig. 3b

Fig. 4a

Fig. 4b

Fig. 4c

Fig. 5

Fig. 6

EP 2 146 628 B1

$C_k$

3

24

25

26

Fig. 7

Fig. 8

EP 2 146 628 B1

Fig. 9

EP 2 146 628 B1

Fig. 10

Fig. 11

## EP 2 146 628 B1

**IN DER BESCHREIBUNG AUFGEFÜHRTE DOKUMENTE**

Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.

**In der Beschreibung aufgeführte Patentdokumente**

- US 4958638 A
- US 3993995 A
- US 5727549 A, Suda
- US 5273036 A, Kronberg
- US 6823739 B, Ueno
- US 6491647 B, Bridger
- US 6875176 B, Mourad
- US 6758816 B, Tsuhata
- DE 102005010498